# EUROPEAN PATENT APPLICATION

(11) **EP 2 441 751 A1**
(43) Date of publication of application: **18.04.2012**
(21) Application number: 10786109.8
(22) Date of filing: 03.06.2010
(51) Int. Cl.: C07C 315/02, C07C 317/04, C07B 61/00

(54) **PROCESS FOR PREPARATION OF ALKYL SULFONE COMPOUNDS**

(30) Priority: 09.06.2009 JP 2009137909; 12.06.2009 JP 2009140677
(71) Applicant: Sumitomo Seika Chemicals Co. Ltd., Hyogo 675-0145 (JP)
(72) Inventor: FUSEYA Ichiro, Kako-gun Hyogo 675-0145 (JP); TAKEUCHI Takeshi, Kako-gun Hyogo 675-0145 (JP); LI Chun, Kako-gun Hyogo 675-0145 (JP); KUAD Paul, Kako-gun Hyogo 675-0145 (JP); HIYAMA Takehiro, Kako-gun Hyogo 675-0145 (JP)
(74) Representative: Webster, Jeremy Mark
(86) International application number: PCT/JP2010/059434
(87) International publication number: WO 2010/143578

(57) **Abstract**

An object of the present invention is to provide a method for preparation of an alkyl sulfone compound simply and safely with high yield, and an alkyl sulfone compound.

The present invention is a method for preparing an alkyl sulfone compound represented by formula (2), comprising:
oxidizing an alkyl sulfide compound represented by formula (1) using an oxidizing agent in the presence of a tungstate catalyst:

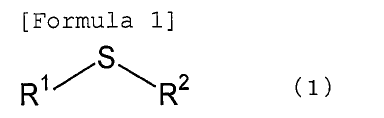

in formula (1), R¹ represents a C1-C3 alkyl group, and R² represents a C3-C5 alkyl group, 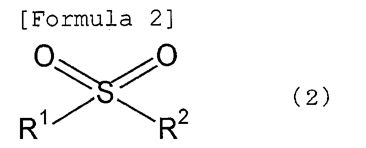 in formula (2), R¹ and R² are the same alkyl groups as R¹ and R² in formula (1), respectively.

## Description

### TECHNICAL FIELD

The present invention relates to a method for preparing an alkyl sulfone compound, and relates to an alkyl sulfone compound.

### BACKGROUND ART

In recent years, aprotic polar solvents such as sulfolane and propylene carbonate have been used as a solvent for an electrochemical device such as a lithium battery and an electric double layer capacitor. In addition to such solvents, acyclic sulfone compounds have generally been used as the aprotic polar solvents.

Examples of a known method for preparing acyclic sulfone compounds known include: a method for oxidizing an alkyl sulfide as a starting material using hydrogen peroxide in an acetic acid solvent (Non-Patent Document 1 and Non-Patent Document 2) and a method for oxidizing an alkyl sulfide as a starting material using t-butylperoxide in a chloroform solvent (Non-Patent Document 3). However, organic solvents and peroxides are used in such methods, and therefore explosive organic peroxides may be generated in a reaction system.

Non-Patent Document 1: Journal of the American Chemical Society (1951), 73, 3627-3632
Non-Patent Document 2: Journal of Organic Chemistry (1946), 11, 475-481
Non-Patent Document 3: Inorganic Chemistry (2002), 41, 1272-1280

### DISCLOSURE OF THE INVENTION

### PROBLEMS TO BE SOLVED BY THE INVENTION

It is an object of the present invention to provide a method for preparing an alkyl sulfone compound simply and safely with high yield, and provide an alkyl sulfone compound.

### MEANS FOR SOLVING THE PROBLEMS

The present invention relates to a method for preparing an alkyl sulfone compound and an alkyl sulfone compound as described below.

A method for preparing an alkyl sulfone compound represented by formula (2), comprising:
oxidizing an alkyl sulfide compound represented by formula (1) using an oxidizing agent in the presence of a tungstate catalyst.

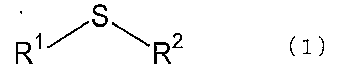

In formula (1), R¹ represents a C1-C3 alkyl group, and R² represents a C3-C5 alkyl group,

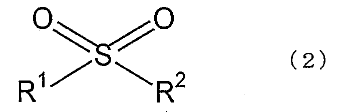

In formula (2), R¹ and R² are the same alkyl groups as R¹and R² in formula (1), respectively.

The method according to claim 1,
wherein the tungstate catalyst is sodium tungstate or potassium tungstate.

The method according to claim 1 or 2,
wherein the oxidizing agent is hydrogen peroxide.

An alkyl sulfone compound used for an electrolyte for an electrochemical device, the alkyl sulfone compound being prepared by a method according to any one of claims 1 to 3.

The alkyl sulfone compound according to claim 4,
wherein the alkyl sulfone compound has a degree of purity of 99% or more and a moisture content of 0.1% by weight or less.

The alkyl sulfide compound represented by formula (1) of the present invention may be one commercially available, or may be prepared by known synthesis methods (Non-Patent Documents 1, 2 and the like) or by the method described below. Particularly, the method described below is preferably used for easily preparing an alkyl sulfide compound with high yield.

That is, an alkylthiol salt represented by formula (4) is prepared by reaction of an alkylthiol represented by formula (3) with a base in the presence of a reducing agent, and the alkylthiol salt is reacted with an alkyl halide represented by formula (5) in the presence of a phase-transfer catalyst. Thus, an alkyl sulfide compound represented by formula (1) can be prepared.

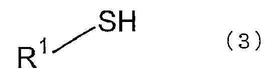

In formula (3), R¹ represents a C1-C3 alkyl group.

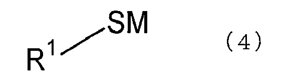

In formula (4), R¹ is the same alkyl group as R¹ in formula (3), and M represents an alkali metal atom.

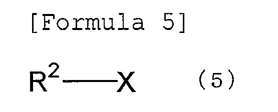

In formula (5), R² represents a C3-C5 alkyl group, and X represents a halogen atom.

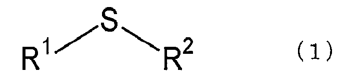

In formula (1), R¹ is the same alkyl group as R¹ in formula (3), and R² is the same alkyl group as R² in formula (5).

Examples of the alkylthiol represented by the formula (3) include methanethiol, ethanethiol, 1-propanethiol, and 2-propanethiol.

Examples of the base used for preparing the alkylthiol salt represented by formula (4) from the alkylthiol in the presence of a reducing agent include: alkali metal hydroxides, such as sodium hydroxide and potassium hydroxide; alkali metal carbonates, such as sodium carbonate and potassium carbonate; and alkali metal alcoholates, such as sodium methylate and sodium ethylate. Particularly, sodium hydroxide is preferably used from the viewpoint of economic efficiency.

The amount of the base is preferably 0.5 to 5.0 moles, and more preferably 0.8 to 2.0 moles, relative to 1 mole of the alkylthiol. When the amount of the base is less than 0.5 mole, the yield of the alkyl sulfide compound may decrease. When the amount of the base is over 5.0 moles, the viscosity of the reaction liquid may increase to cause difficulty in stirring.

Examples of the reducing agent include: sodium borohydride, lithium borohydride, lithium aluminum hydride, sodium cyano borohydride, and diisopropyl aluminum hydride. Particularly, sodium borohydride is preferably used from the availability thereof.

The amount of the reducing agent is preferably 0.001 to 1.0 mole, and more preferably 0.01 to 0.2 mole, relative to 1 mole of the alkylthiol. When the amount of the reducing agent is less than 0.001 mole, an alkyl disulfide compound may generate as an impurity. When the amount of the reducing agent is over 1.0 mole, the viscosity of the reaction liquid may increase to cause difficulty in stirring.

Examples of the alkyl halide represented by the formula (5) include: normal propyl chloride, isopropyl chloride, normal butyl chloride, isobutyl chloride, 1,1-dimethyl ethyl chloride, normal pentyl chloride, 1,2-dimethyl propyl chloride, 2-methylbutyl chloride, 3-methylbutyl chloride, 2,2-dimethyl propyl chloride, normal propyl bromide, isopropyl bromide, normal butyl bromide, isobutyl bromide, 1,1-dimethyl ethyl bromide, normal pentyl bromide, 1,2-dimethyl propyl bromide, 2-methylbutyl bromide, 3-methylbutyl bromide, and 2,2-dimethyl propyl bromide.

The amount of the alkyl halide is preferably 0.5 to 10 moles, and more preferably 0.8 to 5.0 moles, relative to 1 mole of the alkylthiol. When the amount of the alkyl halide is less than 0.5 mole, the reaction may not be completed to cause reduction in yield. When the amount of the alkyl halide is over 10 moles, the degree of purity of the resulting alkyl sulfide compound may decrease.

Examples of the phase-transfer catalyst used for the reaction of the alkylthiol salt represented by the formula (4) with the alkyl halide represented by the formula (5) include: quarternary ammonium salts, such as benzyltriethylammonium bromide, benzyltrimethylammonium bromide, hexadecyltriethylammonium chloride, dodecyltrimethylammonium chloride, tetra-n-butylammonium bromide, tetraethylammonium chloride, and trioctylmethylammonium bromide; and quarternary phosphonium salts, such as a hexadodecyltriethylphosphonium bromide, hexadecyltributylphosphonium chloride, and tetra-n-butylphosphonium chloride. Particularly, tetra-n-butylammonium bromide is preferably used from the viewpoint of increase in yield and the viewpoint of economic efficiency.

The amount of the phase-transfer catalyst is preferably 0.1 to 100 parts by weight, and more preferably 0.1 to 10 parts by weight, relative to 100 parts by weight of alkyl halide. When the amount of the phase-transfer catalyst is less than 0.1 part by weight, the reaction may not be completed to cause reduction in yield. When the amount of the phase-transfer catalyst is over 100 parts by weight, the separation of the catalyst from the product may become difficult to cause reduction in yield of the product.

Examples of a reaction solvent used for the reaction of the alkylthiol salt represented by the formula (4) with the alkyl halide represented by the formula (5) include water, tetrahydrofuran, 1,4-dioxane, methanol, and ethanol. Particularly, ethanol is preferably used from the viewpoint of increase in yield and the viewpoint of economic efficiency.

The amount of the reaction solvent is preferably 10 to 5000 parts by weight, and more preferably 100 to 1000 parts by weight, relative to 100 parts by weight of the alkylthiol. When the amount of the reaction solvent is less than 10 parts by weight, stirring may become difficult to cause reduction in yield. When the amount of the reaction solvent is over 5000 parts by weight, production efficiency may decrease to cause reduction in economic efficiency.

The reaction temperature of the reaction of the alkylthiol salt represented by the formula (4) with the alkyl halide represented by the formula (5) is preferably 30 to 120°C, and more preferably 60 to 110°C. When the reaction temperature is lower than 30°C, the reaction may need to be carried out for a long time for the completion thereof. When the reaction temperature is over 120°C, a side reaction may occur to cause reduction in yield of a target compound. The reaction time is, for example, about 1 to about 30 hours.
The thus prepared alkyl sulfide compound may be rinsed with water and subjected to separation, if necessity. Further, the compound may be isolated with a high degree of purity by distillation.

Specific examples of the alkyl sulfide compound represented by formula (1) used for the present invention include methyl normal propyl sulfide, methylisopropyl sulfide, methyl normal butyl sulfide, methylisobutyl sulfide, methyl 1,1-dimethylethyl sulfide, methyl normal pentyl sulfide, methyl 1,2-dimethylpropyl sulfide, methyl 2-methylbutyl sulfide, methyl 3-methylbutyl sulfide, methyl 2,2-dimethylpropyl sulfide, ethyl normal propyl sulfide, ethylisopropyl sulfide, ethyl normal butyl sulfide, ethylisobutyl sulfide, ethyl 1,1-dimethylethyl sulfide, ethyl normal pentyl sulfide, ethyl 1,2-dimethylpropyl sulfide, ethyl 2-methylbutyl sulfide, ethyl 3-methylbutyl sulfide, ethyl 2,2-dimethylpropyl sulfide, propyl normal propyl sulfide, propylisopropyl sulfide, propyl normal butyl sulfide, propylisobutyl sulfide, propyl 1,1-dimethylethyl sulfide, propyl normal pentyl sulfide, propyl 1,2-dimethylpropyl sulfide, propyl 2-methylbutyl sulfide, propyl 3-methylbutyl sulfide, propyl 2,2-dimethylpropyl sulfide, isopropyl normal propyl sulfide, isopropylisopropyl sulfide, isopropyl normal butyl sulfide, isopropylisobutyl sulfide, isopropyl 1,1-dimethylethyl sulfide, isopropyl normal pentyl sulfide, isopropyl 1,2-dimethylpropyl sulfide, isopropyl 2-methylbutyl sulfide, isopropyl 3-methylbutyl sulfide, and isopropyl 2,2-dimethylpropyl sulfide. Particularly, ethylisopropyl sulfide, ethylisobutyl sulfide, propylisobutyl sulfide, and isopropylisobutyl sulfide are preferably used.

Examples of a tungstate catalyst used for the present invention include sodium tungstate, potassium tungstate, ammonium tungstate, calcium tungstate, iron tungstate, manganese tungstate, and the hydrates thereof. Particularly, sodium tungstate dihydrate and potassium tungstate dihydrate are preferred from the viewpoint of the availability thereof and economic efficiency.

The amount of the tungstate catalyst is preferably 0.01 to 10 parts by weight, and more preferably 0.1 to 10 parts by weight, relative to 100 parts by weight of the alkyl sulfide compound. When the amount of the tungstate catalyst is less than 0.01 part by weight, the reaction may not be completed to cause reduction in yield. When the amount of the tungstate catalyst is over 10 parts by weight, the separation of the catalyst from the product may become difficult to cause reduction in yield of the product.
If the tungstate catalyst is a hydrate, the amount of the tungstate catalyst is converted to the amount of the anhydride thereof.

In the present invention, the alkyl sulfide compound is oxidized using an oxidizing agent in the presence of the tungstate catalyst and an acid, thereby an alkyl sulfone compound is preferably prepared. Examples of the acid include hydrochloric acid, sulfuric acid, nitric acid, perchloric acid, formic acid, acetic acid, carbonic acid, citric acid, and methansulfonic acid. Particularly, hydrochloric acid and sulfuric acid are preferred from the viewpoint of the availability thereof and economic efficiency.

The amount of the acid is preferably 0.01 to 100 parts by weight, and more preferably 0.1 to 100 parts by weight, relative to 100 parts by weight of the alkyl sulfide compound. When the amount of the acid is less than 0.1 part by weight, since the reaction system is not sufficiently acidic, the reaction may not be completed to cause reduction in yield. When the amount of the acid is over 100 parts by weight, the separation of the acid from the product may become difficult to cause reduction in yield of the product.

Examples of the oxidizing agent used in the present invention include hydrogen peroxide, potassium permanganate, potassium chromate, oxygen, 3-chloroperbenzoic acid, and peracetic acid. Particularly, hydrogen peroxide is preferred from the viewpoint of safety and the viewpoint of economic efficiency.

The amount of the oxidizing agent is preferably 1.8 to 10 moles, and more preferably 2 to 5 moles, relative to 1 mole of the alkyl sulfide compound. When the amount of the oxidizing agent is less than 1.8 moles, the reaction may not be completed to cause reduction in yield. When the amount of the oxidizing agent is over 10 moles, no effects matching the amount is exerted and the amount is non-economic.

In the present invention, no reaction solvent is preferably used in order to improve production efficiency and safety, avoid contamination by an organic solvent and/or moisture, and increase the degree of purity of the resulting product. Particularly, a solvent having a low moisture content and a high degree of purity is desirably used as an electrochemical device solvent because device may have problems such as functional decline.

The alkyl sulfone compound having a degree of purity of 99% or more and a moisture content of 0.1% by weight or less can be prepared by the preparation method of the present invention, and therefore such an alkyl sulfone compound is suitable as the solvent for an electrochemical device. When the reaction liquid is too viscous to be sufficiently stirred, a solvent inactive to a reaction may be used, if needed. Examples of the solvent include water, toluene, monochlorobenzene, normal heptane, cyclohexane, dichloromethane, and dichloroethane.

The alkyl sulfide compound represented by the formula (1) is oxidized using an oxidizing agent preferably at a reaction temperature of 0 to 200°C, and more preferably at 10 to 150°C in the presence of the tungstate catalyst. When the reaction temperature is lower than 0°C, the reaction proceeds slowly and may need to be carried out for a long time for the completion thereof. When the reaction temperature is over 200°C, a side reaction may occur to cause reduction in yield of a target compound. The reaction time is generally, for example, 1 to 30 hours.

The thus prepared alkyl sulfone compound is represented by formula (2).

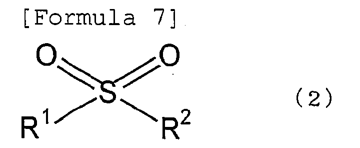

In formula (2), R¹ and R² are the same alkyl groups as R¹ and R² in formula (1), respectively.

The reaction liquid may be rinsed with water and subjected to separation if needed, and the alkyl sulfone compound in accordance with the present invention can be isolated by distillation.

Specific examples of the alkyl sulfone compound represented by formula (2) in accordance with the present invention include: methyl normal propyl sulfone, methylisopropyl sulfone, methyl normal butyl sulfone, methylisobutyl sulfone, methyl 1,1-dimethylethyl sulfone, methyl normal pentyl sulfone, methyl 1,2-dimethylpropyl sulfone, methyl 2-methylbutyl sulfone, methyl 3-methylbutyl sulfone, methyl 2,2-dimethylpropyl sulfone, ethyl normal propyl sulfone, ethylisopropyl sulfone, ethyl normal butyl sulfone, ethylisobutyl sulfone, ethyl 1,1-dimethylethyl sulfone, ethyl normal pentyl sulfone, ethyl 1,2-dimethylpropyl sulfone, ethyl 2-methylbutyl sulfone, ethyl 3-methylbutyl sulfone, ethyl 2,2-dimethylpropyl sulfone, propyl normal propyl sulfone, propylisopropyl sulfone, propyl normal butyl sulfone, propylisobutyl sulfone, propyl 1,1-dimethylethyl sulfone, propyl normal pentyl sulfone, propyl 1,2-dimethylpropyl sulfone, propyl 2-methylbutyl sulfone, propyl 3-methylbutyl sulfone, propyl 2,2-dimethylpropyl sulfone, isopropyl normal propyl sulfone, isopropylisopropyl sulfone, isopropyl normal butyl sulfone, isopropylisobutyl sulfone, isopropyl 1,1-dimethylethyl sulfone, isopropyl normal pentyl sulfone, isopropyl 1,2-dimethylpropyl sulfone, isopropyl 2-methylbutyl sulfone, isopropyl 3-methylbutyl sulfone, and isopropyl 2,2-dimethylpropyl sulfone. Particularly, ethylisopropyl sulfone, ethylisobutyl sulfone, propylisobutyl sulfone, and isopropylisobutyl sulfone are preferred from the viewpoint of excellent characteristics of a solvent for an electrochemical device, and the like.

The alkyl sulfone compound prepared by the preparation method of the present invention is of high purity, and has a low melting point and low viscosity, and is excellent in thermal stability. Therefore, the alkyl sulfone compound is suitable as an electrolyte for an electrochemical device such as a lithium battery, an electric double layer capacitor, a fuel cell, and a dye-sensitized solar cell. Further, the alkyl sulfone compound can be used for a BTX extraction solvent, an acid gas remover, various reaction solvents of an aromatic compound, a photoresist remover, and the like.

### EFFECT OF THE INVENTION

The present invention can provide a method for simply and safety preparing an alkyl sulfone compound suitable for a solvent for an electrochemical device, and the like with high yield, and provide an alkyl sulfone compound.

### MODE FOR CARRYING OUT THE INVENTION

The present invention will, hereinafter, be described more in detail with reference to examples. The present invention is not limited to the examples.

### Synthesis Example 1 [Synthesis of ethylisopropyl sulfide]

A 500-mL four-necked flask equipped with a stirrer, a thermometer, and a condenser was charged with 62.13 g (1.00 mol) of ethanethiol and 1.89 g (0.05 mol) of sodium borohydride under a nitrogen atmosphere. The flask was cooled to 5°C in an ice bath, and charged with 346.67 g (1.30 mol) of a 15% by weight sodium hydroxide aqueous solution. Thus, an aqueous solution of an ethanethiol sodium salt was prepared. A 1000-mL four-necked flask equipped with a stirrer, a thermometer, and a condenser was charged with 135.29 g (1.10 mol) of 2-bromopropane and 32.24 g (0.05 mol) of a 50% by weight tetrabutyl ammonium bromide aqueous solution under a nitrogen atmosphere. The flask was heated to 50°C in a hot water bath, and the total amount of the ethanethiol sodium salt was dropwise added thereto over one hour. The resulting mixture was kept warm for two hours and subjected to separation to give 104.4 g of an organic layer (ethylisopropyl sulfide) which is an upper layer. The yield of the resulting ethylisopropyl sulfide was 100% to the amount of ethanethiol, and the degree of purity thereof was 99.6% determined by gas chromatography analysis.

### Synthesis Example 2 [Synthesis of ethylisobutyl sulfide]

The amount 118.4 g of ethylisobutyl sulfide was prepared in the same manner as in synthesis example 1, except that 150.72 g (1.10 mol) of isobutyl bromide was used instead of 135.29 g of 2-bromopropane in synthesis example 1. The yield of the ethylisobutyl sulfide was 100% to the amount of ethanethiol, and the degree of purity thereof was 95.0% determined by gas chromatography analysis.

### Synthesis Example 3 [Synthesis of propylisobutyl sulfide]

The amount 126.98 g of propylisobutyl sulfide was prepared in the same manner as in synthesis example 1, except that 76.16 g (1.00 mol) of 1-propanethiol was used instead of 62.13 g of ethanethiol in synthesis example 1, and 150.72 g (1.10 mol) of isobutyl bromide was used instead of 135.29 g of 2-bromopropane. The yield of the propylisobutyl sulfide was 96% to the amount of 1-propanethiol, and the degree of purity thereof was 95.6% determined by gas chromatography analysis.

### Synthesis Example 4 [Synthesis of isopropylisobutyl sulfide]

The amount 124.33 g of isopropylisobutyl sulfide was prepared in the same manner as in synthesis example 1, except that 76.16 g (1.00 mol) of 2-propanethiol was used instead of 62.13 g of ethanethiol in synthesis example 1, and 150.72 g (1.10 mol) of isobutyl bromide was used instead of 135.29 g of 2-bromopropane. The yield of the isopropylisobutyl sulfide was 94% to the amount of 2-propanethiol, and the degree of purity thereof was 90.6% determined by gas chromatography analysis.

### Example 1

A 500-mL four-necked flask equipped with a stirrer, a thermometer, and a condenser was charged with 104.2 g (1.00 mol) of ethylisopropyl sulfide prepared in synthesis example 1, and 4.0 g of sodium tungstate dihydrate and 52 g of a 25% by weight sulfuric acid aqueous solution were added thereto under a nitrogen atmosphere. The flask was heated to 50°C in a hot water bath, and 194.0 g (2.00 mol) of a 35% by weight hydrogen peroxide solution was subsequently added, and the solution was heated to 60°C and reacted with stirring for two hours. After the completion of the reaction, 7.19 g of sodium sulfite was added to the resulting mixture, and 30.7 g of a 30% by weight sodium hydroxide aqueous solution was added. The resulting solution was stirred and subjected to separation to remove the water layer. The resulting organic layer was subjected to simple distillation, thereby 122.6 g of clear and colorless ethylisopropyl sulfone in liquid form was prepared. The yield of the ethylisopropyl sulfone was 90% to the amount of ethylisopropyl sulfide, and the degree of purity thereof was 99.9% determined by gas chromatography analysis. The moisture content was 0.03% determined by weight by Karl Fischer analysis.

The obtained ethylisopropyl sulfone was measured for the melting point and the exothermic onset temperature using a differential scanning calorimeter under a nitrogen atmosphere. Further, the viscosity thereof was determined using a rotational viscometer (product of Tokimec Inc., trade name "DISITAL VISCOMETER").

### Example 2

The amount 130.7 g of ethylisobutyl sulfone was prepared in the same manner as in example 1, except that 118.2 g of ethylisobutyl sulfide prepared in synthesis example 2 was used instead of 104.2 g of ethylisopropyl sulfide in example 1. The yield of the ethylisobutyl sulfone was 87% to the amount of ethylisobutyl sulfide, and the degree of purity thereof was 99.9% determined by gas chromatography analysis. The moisture content was 0.04% by weight, determined by Karl Fischer analysis.

The obtained ethylisobutyl sulfone was measured for the melting point, exothermic onset temperature, and viscosity by the same method as that in example 1.

### Example 3

The amount 142.91 g of propylisobutyl sulfone was prepared in the same manner as in example 1, except that 132.27 g of propylisobutyl sulfide prepared in synthesis example 3 was used instead of 104.2 g of ethylisopropyl sulfide in example 1. The yield of the propylisobutyl sulfone was 87% to the amount of propylisobutyl sulfide, and the degree of purity thereof was 99.3% determined by gas chromatography analysis. The moisture content was 0.02% determined by weight by Karl Fischer analysis.

The obtained propylisobutyl sulfone was measured for the melting point, exothermic onset temperature, and viscosity by the same method as that in example 1.

### Example 4

The amount 121.56 g of isopropylisobutyl sulfone was prepared in the same manner as in example 1, except that 132.27 g of isopropylisobutyl sulfide prepared in synthesis example 4 was used instead of 104.2 g of ethylisopropyl sulfide in example 1. The yield of the isopropylisobutyl sulfone was 74% to the amount of isopropylisobutyl sulfide, and the degree of purity thereof was 99.7% determined by gas chromatography analysis. The moisture content was 0.04% determined by weight by Karl Fischer analysis.

### Example 5

The amount 123.95 g of ethylisopropyl sulfone was prepared in the same manner as in example 1, except that 4.0 g of potassium tungstate was used instead of 4.0 g of sodium tungstate dihydrate. The yield of the isopropylisobutyl sulfone was 91% to the amount of ethylisopropyl sulfide, and the degree of purity thereof was 99.9% determined by gas chromatography analysis. The moisture content was 0.01% determined by weight by Karl Fischer analysis.

The obtained isopropylisobutyl sulfone was measured for the melting point, exothermic onset temperature, and viscosity by the same method as that in example 1.

### Comparative Example 1

A 500-mL four-necked flask equipped with a stirrer, a thermometer, and a condenser was charged with 104.2 g (1.00 mol) of ethylisopropyl sulfide prepared in synthesis example 1 and 400 g of acetic acid under a nitrogen atmosphere. The flask was heated to 50°C in a hot water bath. Then, 194.0 g (2.00 mol) of a 35% hydrogen peroxide solution was added, and the solution was stirred at 25°C for 48 hours and at 100°C for four hours. After the completion of the reaction, the resulting solution was subjected to simple distillation, thereby 95.0 g of clear and colorless ethylisopropyl sulfone in liquid form was prepared. The yield of the ethylisopropyl sulfone was 80% to the amount of ethylisopropyl sulfide, and the degree of purity thereof was 97.8% determined by gas chromatography analysis. The moisture content was 0.51% determined by weight by Karl Fischer analysis.

### Comparative Example 2

The amount 130.7 g of ethylisobutyl sulfone was prepared in the same manner as in comparative example 1, except that 118.2 g of ethylisobutyl sulfide prepared in synthesis example 2 was used instead of 104.2 g of ethylisopropyl sulfide in comparative example 1. The yield of the ethylisobutyl sulfone was 87% to the amount of ethylisobutyl sulfide, and the degree of purity thereof was 97.5% determined by gas chromatography analysis. The moisture content was 0.23% determined by weight by Karl Fischer analysis.

Table 1 shows the measurement results of the melting point, exothermic onset temperature, and viscosity in accordance with examples 1 to 4, and also shows the measurement results thereof for sulfolane and propylene carbonate for comparison.

**[Table 1]**

| Compound | Melting point (°C) | Exothermic onset temperature (°C) | Viscosity (cp) |
|---|---|---|---|
| Ethylisopropyl sulfone (Example 1) | -15 | 281 | 4(25°C) |
| Ethylisobutyl sulfone (Example 2) | -22 | 233 | 4(25°C) |
| Propylisobutyl sulfone (Example 3) | 8 | 252 | 7(25°C) |
| Isopropylisobutyl sulfone (Example 4) | -20 | 234 | 7(25°C) |
| Sulfolane | 29 | 210 | 10(30°C) |
| Propylene carbonate | -49 | 73 | 3(25°C) |

Examples 1 and 5 were compared to comparative example 1, and example 2 was compared to comparative example 2.
The result is that the alkyl sulfone compound in accordance with the present invention is of high purity and has a low moisture content. Characteristics shown in Table 1 show that the alkyl sulfone compound of the present invention has a low melting point and low viscosity and is excellent in thermal stability because an exothermic onset temperature is high.

### INDUSTRIAL APPLICABILITY

According to the present invention, a method for simply and safety preparing an alkyl sulfone compound with high yield and an alkyl sulfone compound are provided.

## Claims

1. A method for preparing an alkyl sulfone compound represented by formula (2), comprising:
oxidizing an alkyl sulfide compound represented by formula (1) using an oxidizing agent in the presence of a tungstate catalyst: 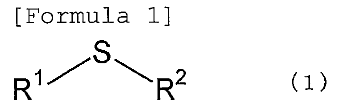 in formula (1), R¹ represents a C1-C3 alkyl group, and R² represents a C3-C5 alkyl group, 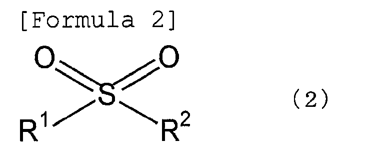 in formula (2), R¹ and R² are the same alkyl groups as R¹ and R² in formula (1), respectively.

2. The method according to claim 1,
wherein the tungstate catalyst is sodium tungstate or potassium tungstate.

3. The method according to claim 1 or 2,
wherein the oxidizing agent is hydrogen peroxide.

4. An alkyl sulfone compound used for an electrolyte for an electrochemical device, the alkyl sulfone compound being prepared by a method according to any one of claims 1 to 3.

5. The alkyl sulfone compound according to claim 4,
wherein the alkyl sulfone compound has a degree of purity of 99% or more and a moisture content of 0.1% by weight or less.
